# EUROPEAN PATENT SPECIFICATION

(11) **EP 3 362 796 B1**
(45) Date of publication and mention of the grant of the patent: **05.02.2020**
(21) Application number: 16791426.6
(22) Date of filing: 16.10.2016
(51) Int. Cl.: G01N 33/574, G01N 33/52

(54) **MUCOSA ANALYSIS**
SCHLEIMHAUTANALYSE
ANALYSE DE LA MUQUEUSE

(30) Priority: 16.10.2015 FI 20155736
(43) Date of publication of application: 22.08.2018
(73) Proprietor: Aqsens Health Oy, 20520 Turku (FI)
(72) Inventor: SIIVONEN, Joonas, 20540 Turku (FI); TIITTANEN, Satu, 20750 Turku (FI)
(74) Representative: Finnpatent Oy
(86) International application number: PCT/FI2016/050721
(87) International publication number: WO 2017/064371

(56) References cited:
- WO-A1-2010/128203
- WO-A1-2013/160547
- WO-A1-2015/017692
- HSIN-MING CHEN ET AL: "Time-resolved autofluorescence spectroscopy for classifying normal and premalignant oral tissues", LASERS IN SURGERY AND MEDICINE., vol. 37, no. 1, 1 January 2005 (2005-01-01), pages 37-45, XP055335162, US ISSN: 0196-8092, DOI: 10.1002/lsm.20192
- SERVEUS L ET AL: "TIME-RESOLVED FLUORESCENCE IMAGING OF EUROPIUM CHELATE LABEL IN IMMUNOHISTOCHEMISTRY AND IN SITU HYBRIDISATION", CYTOMETRY, ALAN LISS, NEW YORK, US, vol. 13, 1 January 1992 (1992-01-01), pages 329-338, XP008075194, ISSN: 0196-4763, DOI: 10.1002/CYTO.990130402

## Description

### FIELD

The present invention is related to a method for determining the presence or absence mucosa alterations in particular to a method including treating an mucosal rinse with a reagent comprising a lanthanide(III) ion and an indicator molecule The invention relates also to an array for determining mucosa alterations.

### BACKGROUND

Each year 600,000 people worldwide are diagnosed with head and neck squamous cell carcinoma (HNSCC). Since oral cavity cancer is the most common site of HNSCC, and the mouth is easily accessible, one might expect screening examinations to be useful in early detection. There exist various commercial diagnostic kits for oral cancer. Although these tests can assist in the identification of abnormalities, they require an experienced professional to interpret the results and follow up for a more definite test such as biopsy.

WO2015017692 discloses a method of determining a risk of cancer in a subject by determining whether the test amount of solCD44 and the test amount of total protein are above or below reference levels of solCD44 and total protein. According to the disclosure, the solCD44 level and the total protein level may be measured from an oral rinse by using immunoassay and UV spectrophotometry. The method is preferably accompanied by a questionnaire in order to take into account e.g. patient risk factors. Although the method disclosed therein is suitable for cancer risk determination, it may still be quite laborious and time consuming especially for use for early detection of alterations in mucosa, in particular in developing countries.

WO2010128203 A1 discloses a method for characterizing and/or determining a sample and an array of non-specific interacting means for characterizing and/or determining a sample and the use of the array.

WO2013160547 A1 discloses a method for characterizing and/or determining samples in the aid of lanthanide(III) ions and ligands including an aromatic structure and 2-5 chelating heteroatoms. The document discloses also an array for characterizing and/or determining samples utilizing the properties of lanthanide(III) chelates.

Chen H.-M. et al (Lasers in Surgery and Medicine 37:37-45, 2005) discloses use of time-resolved autofluorescence spectroscopy for classifying normal and premalignant oral tissues.

WO2015017692 A1 discloses compositions and methods for identifying a risk of cancer in a subject.

### SUMMARY

The present invention is based on the observation that mucosa alterations can be determined simply by admixing a sample obtained from a subject with a reagent comprising a lanthanide(III) ion and an indicator molecule, and determining a signal derived from the lanthanide(III) by luminescence measurement followed by comparison to a signal derived from the sample of the subject with a reference signal derived from samples of healthy subjects and subjects having mucosa alterations.

According to one aspect the present invention concerns a method for determining mucosa alterations in a sample obtained from a subject employing an array for at least two different interacting substances, at least one of which comprises one or more indicator molecules selected from pH indicator dye, ionochromic dye and tissue stain interacting with the sample, wherein the interacting is specific, the method comprising following steps:
a) introducing a solution comprising the sample to the array, wherein the solution or the array comprises a reagent comprising a lanthanide(III) ion, to form at least two admixtures,
b) exciting the at least two admixtures at a first excitation wavelength, and detecting a signal deriving from the lanthanide(III) ion at a first emission wavelength by using luminescent measurement, to obtain a fingerprint of the sample;
c) determining the presence or absence of the mucosa alterations by comparing the fingerprint of the sample with
   i) at least one fingerprint of an array obtained from a sample with mucosa alterations, and/or
   ii) at least one fingerprint of an array obtained from a sample of with no mucosa alterations,
   *in proviso* that the reagent comprising a lanthanide(III) ion does not comprise any sample specific recognition elements or any indicator molecule specific recognition elements.

According to another aspect the present invention concerns an array for determining mucosa alterations in a sample, the array comprising least two different interacting substances, wherein
(i) at least one of the least two different interacting substances comprises one or more indicator molecules configured to interact with the mucosa alterations, wherein the indicator molecule is selected from pH indicator dye, ionochromic dye, and tissue stain, and
(ii) the least two different interacting substances comprise a reagent comprising a lanthanide(III) ion, *in proviso* that the reagent comprising a lanthanide(III) ion does not comprise any sample specific recognition elements or any indicator molecule specific recognition elements.

Further aspects of the present invention are disclosed in the dependent claims.

Exemplifying and non-limiting embodiments of the invention, both as to constructions and to methods of operation, together with additional objects and advantages thereof, are best understood from the following description of specific exemplifying embodiments when read in connection with the accompanying drawings.

The verbs "to comprise" and "to include" are used in this document as open limitations that neither exclude nor require the existence of unrecited features. The features recited in the accompanied depending claims are mutually freely combinable unless otherwise explicitly stated. Furthermore, it is to be understood that the use of "a" or "an", i.e. a singular form, throughout this document does not exclude a plurality.

### BRIED DESCRIPTION OF DRAWINGS

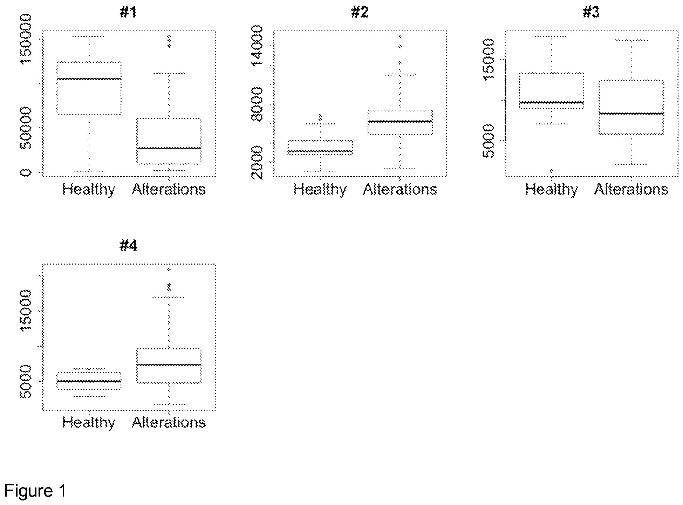

Figure 1 shows exemplary results of the method of the present invention for determining mucosa alterations.

### DESCRIPTION

The present invention concerns a method for determining the presence or absence of mucosa alterations. The method includes determining mucosa alterations in a sample obtained from a subject employing an array of at least two different interacting substances, at least one of which comprises one or more indicator molecules interacting with the sample as defined in the claims.

According one instance, a disclosed method comprises following steps:
a) introducing a sample provided from a subject with a reagent comprising a lanthanide(III) ion to form a first admixture;
b) introducing the first admixture to the at least two interacting substances of the array to form second admixtures;
c) exciting the second admixtures at a first excitation wavelength, and detecting a signal deriving from the lanthanide(III) ion at a first emission wavelength by using luminescent measurement, to obtain a fingerprint of the sample;
d) determining the presence or absence of the mucosa alterations in the sample by comparing the fingerprint with
   i) at least one fingerprint of an array obtained from samples with mucosa alterations, and/or
   ii) at least one fingerprint of an array obtained from a samples of with no mucosa alterations,
   *in proviso* that the reagent comprising a lanthanide(III) ion does not comprise sample or indicator molecule specific recognition elements.

According to another disclosed instance a disclosed method includes determining mucosa alterations in a sample obtained from a subject employing an array of at least two different interacting substances and a reagent comprising a lanthanide(III) ion, at least one of the interacting substances comprising one or more indicator molecules configured to interact with the sample, the method comprises the following steps:
a) introducing a sample provided from a subject to the array to obtain at least two admixtures;
b) exciting the admixtures at a first excitation wavelength, and detecting a signal deriving from the lanthanide(III) ion at a first emission wavelength by using luminescent measurement to obtain a fingerprint of the sample;
c) determining the presence or absence of the mucosa alterations by comparing the fingerprint with
   i) at least one fingerprint of an array obtained from samples with mucosa alterations, and/or
   ii) at least one fingerprint of an array obtained from a samples of with no mucosa alterations,
   *in proviso* that the reagent comprising a lanthanide(III) ion does not comprise sample or indicator molecule specific recognition elements.

A mucous membrane or mucosa is a lining of mostly endodermal origin. It consists of an epithelium (a layer, or layers of epithelial cells) and an underlying lamina propria of loose connective tissue. Mucosae line various cavities of the body that are either externally exposed to the environment or are internal organs, and the mucous membranes ensure that the underlying lamina propria of connective tissue remains moist. They are at several places contiguous with skin: at the nostrils, the lips of the mouth, the eyelids, the ears, the trachea, the stomach, the genital area, and the anus. Exemplary mucosae are ronchial mucosa, endometrium, esophageal mucosa, gastric mucosa, intestinal mucosa, nasal mucosa, olfactory mucosa, oral mucosa, penile mucosa, and vaginal mucosa. According to a preferable embodiment the mucosa is oral mucosa.

As defined herein, an alteration is any abnormality in the tissue of an organism. If an alteration is caused by a tumor it is classified as malignant or benign.

According to a preferable embodiment the method is used for determining alterations in oral mucosa. According to this embodiment the sample is selected from the group consisting of oral rinse, saliva, and sputum. A preferable bodily fluid is oral rinse. If required, the sample can be purified or diluted prior to analysis. Exemplary purification methods are chromatography and filtration.

The sample is admixed with a reagent comprising a lanthanide(III) ion. The lanthanide is preferably selected from europium, terbium, samarium and dysprosium, preferably from europium and terbium. The most preferable lanthanide is europium.

The reagent comprising lanthanide ion is preferably a luminescent lanthanide(III) chelate. Exemplary luminescent lanthanide(III) chelates comprising a pyridine subunit as a part of the chromophore moiety have been disclosed in Bioconjugate Chem. 2009, 20, 404. An exemplary luminescent lanthanide(III) chelate is a terpyridine-Eu(III) (1)

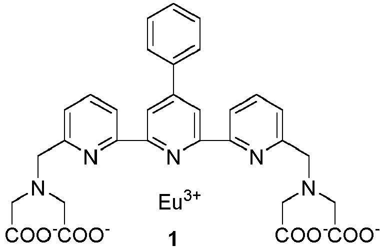

The reagent comprising a lanthanide(III) ion is allowed to chelate and/or coordinate with the sample including one or more sample substances, such as mucous proteins. The reagent comprising a lanthanide(III) ion does not have any sample specific recognition elements and thus it is able to coordinate and/or chelate with the sample non-specifically.

A solution including the sample and a reagent comprising a lanthanide(III) ion is admixed with an array comprising one or more indicator molecules. The indicator molecule is configured to interact with the sample, sample coordinated and/or chelated with reagent comprising a lanthanide(III) ion and/or with the reagent comprising a lanthanide(III) ion. According to the invention, interaction of the indicator with the reagent comprising a lanthanide(III) ion is non-specific, while interaction of the indicator with the sample substance is specific. The term non-specific interaction means that binding or other interaction is not predetermined.

The array comprises the one or more indicator molecules and the reagent comprising a lanthanide(III) ion, and the sample is admixed with the array to give rise to two or more admixtures, preferably two or more solutions comprising the indicator molecules, the sample, and the reagent comprising a lanthanide(III) ion.

The indicator molecules are preferably selected from pH indicator dyes, ionochromic dyes and tissue stains. Exemplary pH indicator dyes are bromothymol blue, phenyl red, methyl red, litmus, phenolphthalein, neutral red, cresol red, bromocresol blue. Exemplary ionochromic dyes are Ca dyes EGTA, BAPTA, fura-2, indo-1, murexide, eriochrome black T, alizarin red S, antipyrylazo III, Mg dyes xylidyl blue, bromopyrogallol red, calconcarboxylic red, calmagite, Cd dyes 2-mercaptobenzothiazole and arsenazo III and dyes for other metals 1-(2pyridylazo)-2-naphthol, 4-(2-pyridylazo)resorcinol, pyrocatechol violet, 2,6-Bis{[bis(2-pyridylmethyl)amino]methyl}-4-methylphenol, zincon monosodium salt. Exemplary tissue stains are methylene blue, toluidine blue O, brilliant cresyl blue, Wright's stain, eosin B, anilin blue, acid orange, crystal violet, acridine orange, Safranin O, and Tartazin. Exemplary other suitable dyes are malachite green, new fuchsin, and rhodamine 800.

The lanthanide(III) ion is detected preferably using time-gated luminescence. Any TRF reader can be employed. Excitation and emission wavelengths can be selected so that the S/N is the best. Also the delay time can be optimized. According to an exemplary instance the lanthanide(III) ion is europium and the excitation and emission wavelength is 395 nm and 615 nm, respectively. An exemplary delay time for europium is 400 µs. When other lanthanides, i.e. terbium, samarium or dysprosium are used, the excitation and emission wavelengths and the delay time are chosen based on the requirements of the lanthanide ion.

The presence or absence of mucosa alterations is determined by comparison with a reference fingerprints obtained from samples of population of healthy individuals and samples of population of individuals including mucosa alterations. Typical results are shown in Figure 1. Accordingly, the present method is able to separate samples including mucosa alterations from mucosa samples with no alterations.

### EXPERIMENTAL

### A typical procedure

Mouth of a subject was rinsed with 20-25 ml of physiological salt solution. Sample was then collected and stored in a freezer if not analyzed immediately. Eu-terpyridine chelate (1) was admixed with the sample to give a chelate concentration of 0.76 nM. A 70 µL of the admixture was dispersed to a microtiter plate containing the dyes. After a 10 min incubation at ambient temperature, the signal derived from the europium(III) ion was measured with Labrox plate reader (excitation wavelength 340 nm; emission wavelength. 615 nm). Final concentrations and exemplary dye compositions are collected in Table 1.

**Table 1.**

| **#** | **Indicator** | **c(**µ**M)** | **c(**µ**M)** | **Dye sensitivity** |
|---|---|---|---|---|
| 1 | o-cresolphtalein complexone | 1006 | | salts/metal ions |
| 2 | EGTA + EosinB | 50 | 52 | proteins |
| 3 | EGTA+ pyrocathecol violet | 50 | 101 | salts |
| 4 | 3-HPA+ bromocresol purple | 50 | 71 | pH/albumin |

The measurement results are shown in Figure 1. The data was divided to a separate teaching and testing sets. The presence of alterations in the measured fingerprints was predicted for the test set by using a k-nearest neighbor's algorithm trained with the training set. The accuracy of the prediction of alterations was 90 % for the test set. The algorithm is able to differentiate the samples of healthy subjects and samples of subjects having mucosa alterations.

The specific examples provided in the description given above should not be construed as limiting the scope and/or the applicability of the appended claims.

## Claims

1. A method for determining mucosa alterations in sample employing an array for at least two different interacting substances, at least one of which comprises one or more indicator molecules selected from pH indicator dye, ionochromic dye and tissue stain interacting with the sample, wherein the interacting is specific, the method comprising following steps:
a) introducing a solution comprising the sample to the array, wherein the solution or the array comprises a reagent comprising a lanthanide(III) ion, to form at least two admixtures,
b) exciting the at least two admixtures at a first excitation wavelength, and detecting a signal deriving from the lanthanide(III) ion at a first emission wavelength by using luminescent measurement to obtain a fingerprint of the sample;
c) determining the presence or absence of the mucosa alterations by comparing the fingerprint of the sample with
i) at least one fingerprint of an array obtained from a sample with mucosa alterations, and/or
ii) at least one fingerprint of an array obtained from a sample of with no mucosa alterations,
*in proviso* that the reagent comprising a lanthanide(III) ion does not comprise any sample specific recognition elements or any indicator molecule specific recognition elements.

2. The method according to claim 1, wherein the reagent comprising a lanthanide ion is a luminescent lanthanide(IIII) chelate.

3. The method according to claim 1 or 2, wherein the mucosa is oral mucosa.

4. The method according to claim 3, wherein the sample is oral rinse.

5. The method according to any of claims 1-4 wherein the alterations are related to cancer, in particular to head and neck squamous cell carcinoma (HNSCC).

6. An array for determining mucosa alterations in a sample, the array comprising least two different interacting substances, wherein
(i) at least one of the least two different interacting substances comprises one or more indicator molecules configured to interact with the mucosa alterations wherein the indicator molecule is selected from pH indicator dye, ionochromic dye, and tissue stain, and wherein
(ii) the least two different interacting substances comprise a reagent comprising a lanthanide(III) ion, *in proviso* that the reagent comprising a lanthanide(III) ion does not comprise any sample specific recognition elements or any indicator molecule specific recognition elements.

7. The array according to claim 6, wherein the reagent comprising a lanthanide ion is a luminescent lanthanide(IIII) chelate.

## Patentansprüche

1. Verfahren zum Bestimmen von Schleimhautveränderungen in einer Probe unter Verwendung einer Anordnung für mindestens zwei verschiedene wechselwirkende Substanzen, von denen mindestens eine ein oder mehrere Indikatormoleküle umfasst, das bzw. die aus einem pH-Indikatorfarbstoff, einem ionochromen Farbstoff und einem Gewebefarbstoff, die mit der Probe wechselwirken, ausgewählt ist bzw. sind, wobei die Wechselwirkung spezifisch ist, wobei das Verfahren die folgenden Schritte umfasst:
a) Einbringen einer Lösung umfassend die Probe zu der Anordnung, wobei die Lösung oder die Anordnung ein Reagenz umfasst, das ein Lanthanid(III)-Ion umfasst, um mindestens zwei Beimischungen zu bilden,
b) Anregen der mindestens zwei Beimischungen bei einer ersten Anregungswellenlänge und Detektieren eines von dem Lanthanid(III)-Ion stammenden Signals bei einer ersten Emissionswellenlänge unter Verwendung von Lumineszenzmessung, um einen Fingerabdruck der Probe zu erhalten;
c) Bestimmen des Vorhandenseins oder Nichtvorhandenseins der Schleimhautveränderungen durch Vergleichen des Fingerabdrucks der Probe mit
i) mindestens einem Fingerabdruck einer Anordnung, die von einer Probe mit Schleimhautveränderungen erhalten wurde, und/oder
ii) mindestens einem Fingerabdruck einer Anordnung, die von einer Probe ohne Schleimhautveränderungen erhalten wurde,
mit der Maßgabe, dass das Reagenz, das ein Lanthanid(III)-Ion umfasst, keine probenspezifischen Erkennungselemente oder indikatormolekülspezifischen Erkennungselemente umfasst.

2. Verfahren nach Anspruch 1, wobei das Reagenz umfassend ein Lanthanid-Ion ein lumineszierendes Lanthanid(IIII)chelat ist.

3. Verfahren nach Anspruch 1 oder 2, wobei die Schleimhaut Mundschleimhaut ist.

4. Verfahren nach Anspruch 3, wobei die Probe ein Mundspülung ist.

5. Verfahren nach einem der Ansprüche 1 bis 4, wobei die Veränderungen mit Krebs zusammenhängen, insbesondere mit Kopf- und Hals-Plattenepithelkarzinomen (HNSCC).

6. Anordnung zum Bestimmen von Schleimhautveränderungen in einer Probe, wobei die Anordnung mindestens zwei verschiedene wechselwirkende Substanzen umfasst, wobei
(i) mindestens eine der mindestens zwei verschiedenen wechselwirkenden Substanzen ein oder mehrere Indikatormoleküle umfasst, das bzw. die zur Wechselwirkung mit den Schleimhautveränderungen konfiguriert ist bzw. sind, wobei das Indikatormolekül aus einem pH-Indikatorfarbstoff, einem ionochromen Farbstoff und einem Gewebefarbstoff ausgewählt ist, und wobei
(ii) die mindestens zwei verschiedenen wechselwirkenden Substanzen ein Reagenz umfassen, das ein Lanthanid(III)-Ion umfasst, mit der Maßgabe, dass das Reagenz, das ein Lanthanid(III)-Ion umfasst, keine probenspezifischen Erkennungselemente oder indikatormolekülspezifischen Erkennungselemente umfasst.

7. Anordnung nach Anspruch 6, wobei das Reagenz umfassend ein Lanthanid-Ion ein lumineszierendes Lanthanid(IIII)chelat ist.

## Revendications

1. Procédé de détermination de modifications de la muqueuse dans un échantillon employant un réseau pour au moins deux substances interagissant différentes, dont au moins une comprend une ou plusieurs molécules indicatrices choisies parmi un colorant indicateur de pH, un colorant ionochromique et une teinte de tissu interagissant avec l'échantillon, où l'interaction est spécifique, le procédé comprenant les étapes suivantes consistant à :
a) introduire une solution comprenant l'échantillon au réseau, où la solution ou le réseau comprend un réactif comprenant un ion lanthanide (III), pour former au moins deux mélanges,
b) exciter les au moins deux mélanges à une première longueur d'onde d'excitation, et détecter un signal dérivé de l'ion lanthanide (III) à une première longueur d'onde d'émission en utilisant une mesure luminescente pour obtenir une empreinte de l'échantillon ;
c) déterminer la présence ou l'absence des modifications de la muqueuse en comparant l'empreinte de l'échantillon avec
i) au moins une empreinte d'un réseau obtenue à partir d'un échantillon avec des modifications de muqueuse, et/ou
ii) au moins une empreinte d'un réseau obtenue d'un échantillon sans modifications de muqueuse
*à condition que* le réactif comprenant un ion lanthanide (III) ne comprenne aucun élément de reconnaissance spécifique d'un échantillon ou aucun élément de reconnaissance spécifique d'une molécule indicatrice.

2. Procédé selon la revendication 1, dans lequel le réactif comprenant un ion lanthanide est un chélate de lanthanide (IIII) luminescent.

3. Procédé selon la revendication 1 ou 2, dans lequel la muqueuse est la muqueuse orale.

4. Procédé selon la revendication 3, dans lequel l'échantillon est un rinçage oral.

5. Procédé selon l'une quelconque des revendications 1 à 4 dans lequel les modifications sont associées à un cancer, en particulier à un carcinome cellulaire squameux de la tête et du cou (HNSCC).

6. Réseau de détermination de modifications de la muqueuse dans un échantillon, le réseau comprenant au moins deux substances interagissant différentes, où
(i) au moins une des deux substances interagissant différentes comprend une ou plusieurs molécules indicatrices configurées pour interagir avec les modifications de la muqueuse où la molécule indicatrice est choisie parmi un colorant indicateur de pH, un colorant ionochromique et une teinte de tissu, et où
(ii) les au moins deux substances interagissant différentes comprennent un réactif comprenant un ion lanthanide (III), *à condition que* le réactif comprenant un ion lanthanide (III) ne comprenne aucun élément de reconnaissance spécifique d'un échantillon ou aucun élément de reconnaissance spécifique d'une molécule indicatrice.

7. Réseau selon la revendication 6, dans lequel le réactif comprenant un ion lanthanide est un chélate de lanthanide (IIII) luminescent.
